# EUROPEAN PATENT APPLICATION

(11) **EP 4 349 266 A1**
(43) Date of publication of application: **10.04.2024**
(21) Application number: 22811165.4
(22) Date of filing: 12.05.2022
(51) Int. Cl.: A61B 8/14, G06T 7/00

(54) **FEATURE EXTRACTION DEVICE, FEATURE EXTRACTION METHOD, PROGRAM, AND INFORMATION RECORDING MEDIUM**

(30) Priority: 28.05.2021 JP 2021089721
(71) Applicant: RIKEN, Wako-shi, Saitama 351-0198 (JP)
(72) Inventor: YAMAMOTO, Yoichiro, Wako-shi, Saitama 351-0198 (JP)
(74) Representative: Epping - Hermann - Fischer
(86) International application number: PCT/JP2022/020038
(87) International publication number: WO 2022/249892

(57) **Abstract**

A feature extraction device (101) extract features of a target from a plurality of images related to the target. The image processor (111) calculates, using an image model (151), a likelihood of the inputted image belonging to a first image class, and a feature parameter of the inputted image. A feature processor (112) that inputs an image included in the inputted image group into the image processor (111) to calculate the likelihood and the feature parameter, selects, based on the calculated likelihood, a predetermined number of representative images from the inputted image group, and outputs, as feature information of the image group, the feature parameter calculated for the selected predetermined number of representative images. A classification processor (113) is an additional element, inputs a target image group related to a target to the feature processor (112), and predicts, from the outputted feature information and using a classification model, whether the target belongs to a first target class. Here, an image belonging to the first image class corresponds to the target belonging to the first target class.

## Description

### Technical Field

The present disclosure relates to a feature extraction device, a feature extraction method, a program, and an information recording medium that extract features of a target from a plurality of images related to the target.

### Background Art

In the related art, technologies have been proposed that seek to extract features by processing, using a neural network, photographs in which a target is captured, classify the target on the basis of the features, and utilize the results in various uses including medical diagnosis.

For example, Patent Literature 1 describes technology for receiving a target image in which a target is captured and one or more attribute parameters associated with the target and, when classifying the target using a neural network, convolving each element of a provided feature map and the received one or more attribute parameters.

Meanwhile, when obtaining a photograph by imaging, ultrasonically or the like, a site of a subject that is the target of an examination, multiple images may be acquired for one target. Moreover, when multiple organs with different functions are captured in a single photograph, this single photograph may be divided into multiple small images to enable processing for every site.

In such a case, it is thought that, even for patients with lesions or the like, multiple images will exist in which regions are captured that are indistinguishable from those of a healthy person

However, commonly, in a prognostic diagnosis such as prediction of recurrence of prostate cancer or the like, a target site excised from the subject is used as a specimen and, on the basis of medical knowledge, a doctor distinguishes and isolates the cancerous region (lesioned region) from other regions (normal regions) from a pathological photograph taken of the specimen. For example, in Gleason classification, which is widely used to classify cancer malignancy, after distinguishing the cancerous region, the tissue morphology of the cancer is further examined to determine the Gleason score, which indicates the malignancy.

A great deal of effort and time is required to carry out such distinguishing and, in addition to the accuracy of the results varying by doctor, there is a problem in that only appearances that are recognizable by existing medical knowledge can be analyzed.

Moreover, when diagnosing whether or not a subject is suffering from prostate cancer, being able to obtain useful information beneficial for diagnosing from the ultrasound photographs or the like is expected to lead to a reduction in the burden of subsequent tests such as biopsies.

### Citation List

### Patent Literature

Patent Literature 1: Japanese Patent No. 6345332

### Summary of Invention

### Technical Problem

Accordingly, in order to predict whether a target is suffering from a specific disease, the features of the target must be appropriately extracted from multiple images related to the target.

Additionally, in various uses other than the prediction of disease prevalence as well, being able to appropriately extract the features of the subject enables the appropriate classification of the target.

Therefore, there is a need for technology for appropriately extracting, from multiple images related to a target, features of the target that are useful in the classification of the target.

The present disclosure solves the problem described above, and an objective of the present disclosure is to provide a feature extraction device, a feature extraction method, a program, and an information recording medium that extract features of a target from a plurality of images related to the target.

### Solution to Problem

A feature extraction device according to the present disclosure includes:
an image processor that, when an image is input, calculates, using an image model, a likelihood of the inputted image belonging to a first image class, and a feature parameter of the inputted image; and
a feature processor that, when an image group is input, inputs an image included in the inputted image group into the image processor to calculate the likelihood and the feature parameter, selects, based on the calculated likelihood, a predetermined number of representative images from the inputted image group, and outputs, as a feature of a target, the feature parameter calculated for the selected predetermined number of representative images.

### Advantageous Effects of Invention

According to the present disclosure, it is possible to provide a feature extraction device, a feature extraction method, a program, and an information recording medium that extract features of a target from a plurality of images related to the target.

### Brief Description of Drawings

FIG. 1 is an explanatory drawing illustrating an overview of the configuration of a feature extraction device according to an embodiment of the present disclosure;
FIG. 2 is a flowchart illustrating the flow of control of training processing for training an image model;
FIG. 3 is a flowchart illustrating the flow of control of training processing for training a classification model;
FIG. 4 is a flowchart illustrating the flow of control of image processing for obtaining feature information from an image group;
FIG. 5 is a flowchart illustrating the flow of control of feature extraction processing;
FIG. 6 is a flowchart illustrating the flow of control of classification processing;
FIG. 7 is a graph of experiment results of classification according to a conventional method;
FIG. 8 is graph of experiment results of classification according to the embodiment; and
FIG. 9 is an explanatory drawing in which the graph of experiment results of the classification according to the embodiment and the graph of experiment results of the classification according to the conventional method are stacked and compared.

### Description of Embodiments

Hereinafter, embodiments of the present disclosure are described. However, the following embodiments are presented for the purpose of explanation and should not be construed as limiting the scope of the invention of the present disclosure. Therefore, embodiments in which some elements or all elements of these embodiments are replaced with equivalent elements by a person skilled in the art can also be employed. Moreover, the elements described in each example can be appropriately omitted depending on the use. Thus, all embodiments configured in accordance with the principles of the present disclosure are included within the scope of the present disclosure.

### Configuration

The feature extraction device according to the present embodiment is typically realized by a computer executing a program. This computer is connected to various types of output devices and input devices, and exchanges information with these devices.

The program executed by the computer can be distributed or sold by a server to which the computer is communicably connected. In addition, the program executed by the computer can be stored on a non-transitory information recording medium such as a compact disk read only memory (CD-ROM) or an electrically erasable programmable ROM (EEPROM), and this non-transitory information recording medium can be distributed, sold, or the like.

The program is installed on a non-transitory information recording medium such as a hard disk, a solid state drive, a flash memory, an EEPROM, or the like of the computer. Upon installation, an information processing device of the present embodiment is realized by the computer. Typically, under management by an operation system (OS) of the computer, a central processing unit (CPU) of the computer reads the program from the non-transitory information recording medium into random access memory (RAM), and then interprets and executes the code included in the program. However, in an architecture in which the non-transitory information recording medium can be mapped within memory space accessible by the CPU, explicit loading of the program to the RAM may be unnecessary. Note that the various information required in the process of execution of the program can be temporarily stored in the RAM.

Furthermore, as described above, the computer includes a graphics processing unit (GPU), and it is desirable that the computer includes a GPU for carrying out various types of image processing calculations at high speed. Using the GPU and a library such as TensorFlow or PyTorch enables the utilization, under the control of the CPU, of learning functions, classification functions, and the like in various types of artificial intelligence processes.

Note that it is possible to configure the information processing device of the present embodiment using dedicated electronic circuits instead of realizing the information processing device of the present embodiment by a general use computer. In such an aspect, the program can be used as a material for generating timing charts, wiring diagrams, and the like of electronic circuits. Moreover, in such an aspect, electronic circuits that satisfy the specifications stipulated by the program are configured from field programmable gate arrays (FPGA) or application specific integrated circuits (ASIC), and the electronic circuits function as dedicated devices that fulfill functions stipulated by the program to realize the information processing device of the present embodiment.

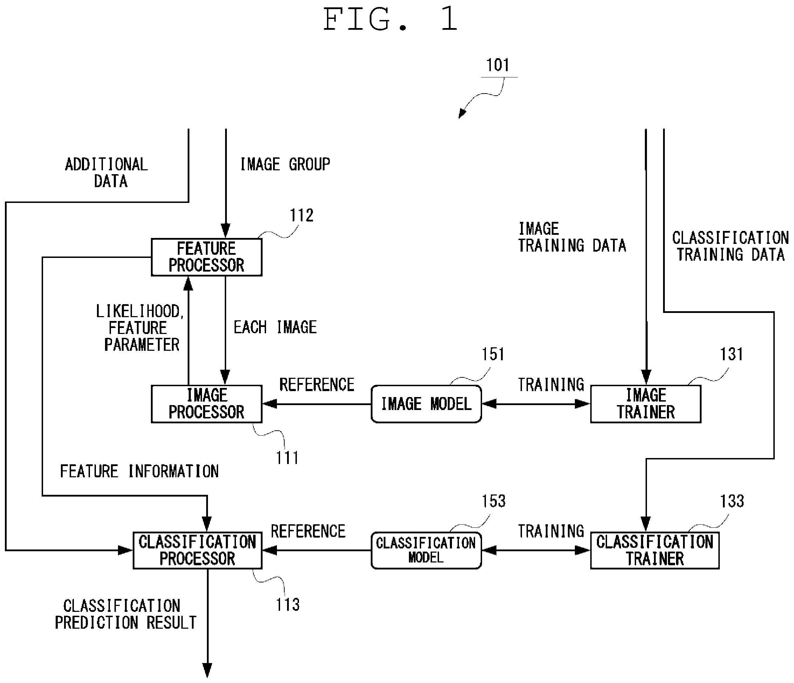

In the following, to facilitate comprehension, a feature extraction device 101 is described assuming an aspect in which the computer is realized by the program being executed. FIG. 1 is an explanatory drawing illustrating an overview of the configuration of the feature extraction device according to an embodiment of the present disclosure.

As illustrated in FIG 1, the feature extraction device 101 according to the present embodiment includes an image processor 111 and a feature processor 112. Additionally, the feature extraction device 101 includes a classification processor 113 as an omittable component.

The image processor 111 references an image model 151. The feature extraction device 101 can include, as an omittable component, an image trainer 131 for training the image model 151. For example, it is possible to omit the image trainer 131 when using, as the image model 151, a model that is already trained.

The feature processor 112 references a classification model 153. The feature extraction device 101 can include, as an omittable component, a classification trainer 133 for training the classification model 153. For example, it is possible to omit the classification trainer 133 when using, as the classification model 153, a model that is already trained.

The image trainer 131 and the classification trainer 133 can also be implemented as devices independent from the feature extraction device 101. In this aspect, a trained parameter constituting the trained image model 151 and/or classification model 153 and a prediction program that uses the trained parameter is transferred to the feature extraction device 101 from the image trainer 131 and/or the classification trainer 133 via a non-transitory information recording medium and/or a computer communication network. Note that, in the present application, to facilitate comprehension, the training of the parameters of models such as the image model 151 and the classification model 153 is sometimes expressed as training, learning, updating, and the like of the model.

When an image is input, the image processor 111 calculates, using the image model 151, a likelihood of the inputted image belonging to a first image class, and a feature parameter of the inputted image. Accordingly, when a plurality of images are sequentially (or in parallel, or as a batch) input into the image processor 111, the likelihood and the feature parameter for each image are sequentially (or in parallel, or as a batch) output from the image processor 111.

Here, a model related to a deep convolutional neural network or the like, or other various types of models can be used as the image model 151.

The image processor 111 calculates the likelihood from a vector of pixel values and, as such, can be thought of as reducing the dimensionality of a vector value. When the image model 151 is related to a neural network or the like, information is exchanged across a plurality of layers to reduce the dimensionality. Information output in an intermediate layer can be used as the feature parameter. That is, an intermediate vector, in the image model 151, for which the dimensionality is in the process of being reduced can be used as the feature parameter.

Most simply, the likelihood related to the image can be used as-is as the feature parameter. That is, the likelihood, which is the ultimate result of the dimensionality reduction in the image model 151, can be used as the feature parameter.

Meanwhile, when an image group is input, the feature processor 112 outputs feature information about that image group.

Firstly, the feature processor 112 inputs, into the image processor 111, the images included in the inputted image group to calculate likelihoods and feature parameters.

Then, the feature processor 112 selects, from the inputted image group, a predetermined number of representative images on the basis of the calculated likelihoods.

Then, the feature processor 112 outputs, as the feature information of the image group, the feature parameters calculated for the selected predetermined number of representative images.

Any number of one or greater can be used as the number of representative images selected for one image group.

For example, when one representative image is selected, the feature information is the feature parameter of that representative image, and when the feature information is the likelihood as-is, the feature information is a scalar value consisting of that likelihood.

When three representative images are selected, the feature information is a vector, a tensor, or an array obtained by arranging the feature parameters of the representative images. When the feature parameters are the likelihoods as-is, the feature information is a vector value obtained by arranging the three likelihoods.

Generally, the feature parameter for one image is a vector of N dimensions and, when M representative images are selected, the feature information output from the feature processor 112 for one image group is a vector of N×M dimensions.

The simplest method for selecting the representative images on the basis of the likelihood involves selecting the predetermined number of representative images in descending order of likelihood.

In this method, the feature information emphasizes a feature, of the image group, that corresponds to the first image class.

Another conceivable method involves selecting the predetermined number of representative images in descending order of the absolute value of the difference between the likelihood and a predetermined reference value. For example, when the likelihood is assumed to be the probability of the image belonging to the first image class, the likelihood is a value from 0 to 1, and the predetermined reference value is a boundary value for determining whether the image belongs to the first image class, and can be set to 0.5.

In this method, the feature information can emphasize, to a greater extent than in the method described above, the contrast of whether or not the image group corresponds to the first image class.

Another method involves selecting, as the predetermined number of representative images, images for which the likelihood is a minimum value, a median value, and a maximum value.

In this method, the feature information emphasizes, to a greater extent than in the method described above, the degree to which the image group is dispersed with respect to the first image class.

When a target image group related to a target is input, the classification processor 113 inputs the inputted target image group into the feature processor 112 to predict, from the feature information output from the feature processor 112 and using the classification model 153, whether the target belongs to a first target class.

When the image group input into the feature processor 112 is an image group including images in which a single common target is captured, the feature information output from the feature processor 112 expresses the relationship between that target and the first image class.

Accordingly, if the first image class and the first target class are set so that an image, included in the target image group related to the target, belonging to the first image class corresponds to the target belonging to the first target class as the first image class, and the feature processor 112 selects the representative images so as to emphasize the features of the image group, the classification of the target can be appropriately performed by using the feature information exported from the feature processor 112.

A configuration is possible in which, at this time, in addition to the target image group related to the target, additional data related to the target is input into the classification processor 113. In such a case, the classification processor 113 inputs the inputted target image group into the feature processor 112 to predict, from the feature information output from the feature processor 112 and the inputted additional data, and using the classification model 153, whether the target belongs to the first target class.

Here, models related to linear regression, logistic regression, ridge regression, lasso regression, or a support vector machine, or the like, or other various types of models can be used as the classification model 153.

In addition, the image trainer 131 uses training data including sets of an image and a label indicating whether that image belongs to the first image class to update the image model 151 and advance the training.

When the feature information of the target image group related to the target and the additional data related to the target are provided, the classification trainer 133 uses training data including sets of that additional data and a label indicating whether that target belongs to the first target class to update the classification model 153 and advance the training.

Hereinafter, an example is described in which the present embodiment is applied to the diagnosis of a prostate. Firstly, the target is a subject or a patient that receives a diagnosis of prostate cancer. Accordingly, the first target class is a class indicating that the target is (has a high possibility of) suffering from prostate cancer.

A plurality of images captured by ultrasound, or a plurality of images obtained by dividing a captured photograph into a predetermined size is used as the target image group.

An age of the target, a prostate specific antigen (PSA) value, a total prostate volume (TPV) value, a PSA density (PSAD) value, and the like can be used as the additional data.

The simplest example of the first image class is a class indicating that the target for which the image is captured is suffering from prostate cancer.

In such a case, as the training data required to advance the training of the image model 151, multiple sets of one image in which the target is captured, and a label indicating whether that target is suffering from prostate cancer or, rather, whether that target belongs to the first target class are prepared.

In the training data of this aspect, the images related to the same target share the same label.

Where there is information about a patient for which prostate cancer was discovered in a past test, or there is information about a subject that received a diagnosis of a possibility of cancer in an ultrasound test, was put under observation based on a biopsy in which a piece of tissue is excised and observed under a microscope, and does not develop symptoms, a class indicating that the Gleason score, assigned to the specimen site corresponding to the image site captured in the image in the biopsy specimen, is greater than or equal to a predetermined value can be used as the first image class.

In such a case, as the training data required to advance the training of the image model 151, image training data including multiple sets are prepared of one image in which the site of the target is captured, and a label indicating whether the Gleason score, assigned to that site on the basis of the biopsy specimen, is greater than or equal to a predetermined value.

In the training data in this aspect, when the sites captured in the images differ, different labels may be assigned even when the images are images related to the same target.

The training of the classification model 153 can be advanced after the training of the image model 151 is ended. In this use example, if it is possible to prepare, as the data required to advance the training of the classification model 153, feature information obtained using the image model 151 from the target image group related to the target (photographic images of the subject captured by ultrasound or the like, and/or images obtained by dividing the captured image into a predetermined size), classification training data will be prepared that includes multiple sets of the additional data such as the age of the target, the PSA value, the TPV value, the PSAD value, and the like and a label indicating the results of a final diagnosis of whether the target is positive for prostate cancer.

### Feature extraction training processing

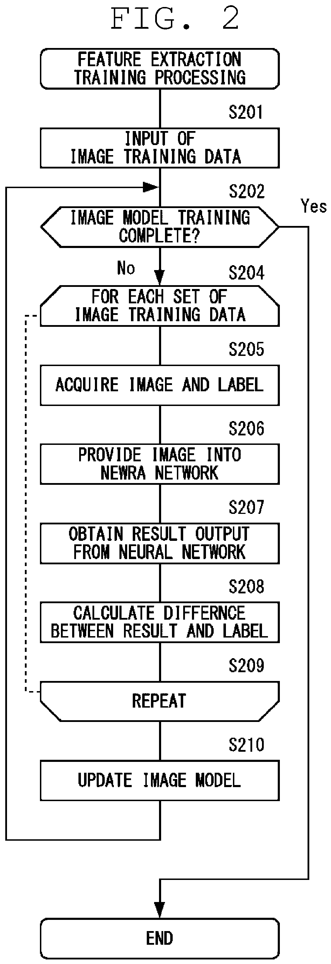

FIG. 2 is a flowchart illustrating the flow of the control of training processing for training the image model. FIG. 2 is referenced in the following description.

When this processing starts, the image trainer 131 firstly receives an input of image training data (step S201).

Then, the image trainer 131 repeats the following processing until the training of the image model 151 is complete (step S202; No).

That is, the image trainer 131 repeats the following processing for each set included in the image training data (step S204).

The image trainer 131 acquires the image and the label included in the set (step S205), provides the acquired image as input into the neural network related to the image model 151 (step S206), obtains a result output from the neural network (step S207), and calculates the difference between the outputted result and the label (step S208).

When the repeating for each set ends (step S209), the image trainer 131 calculates a value of an evaluation function on the basis of the difference calculated for each set and updates the image model 151 (step S210), and executes the processing of step S202.

When the training of the image model 151 is complete (step S202; Yes), the image trainer 131 ends this processing.

### Classification training processing

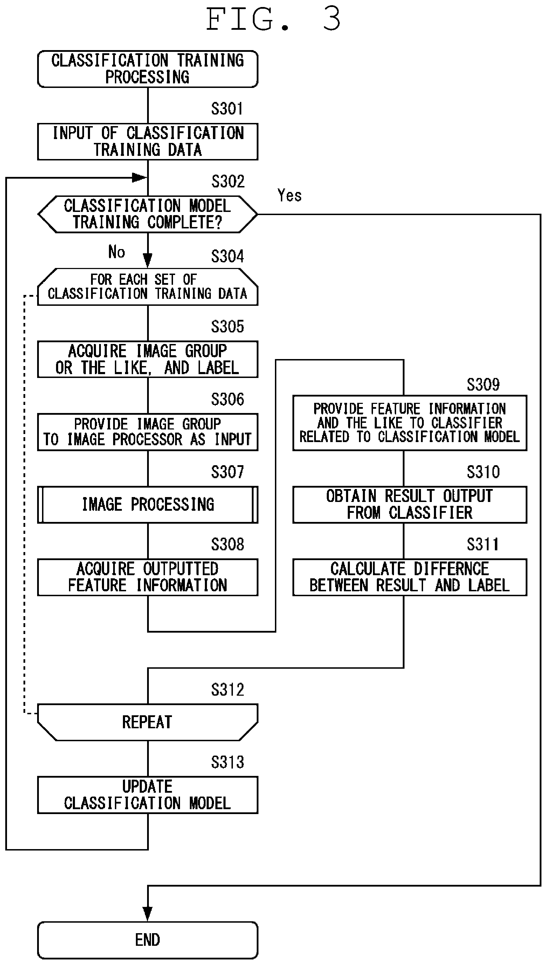

FIG. 3 is a flowchart illustrating the flow of the control of training processing for training the classification model. FIG. 3 is referenced in the following description.

When this processing starts, the classification trainer 133 firstly receives an input of classification training data (step S301).

Then, the image trainer 131 repeats the following processing until the training of the classification model 153 is complete (step S302; No).

That is, the image trainer 131 repeats the following processing for each set included in the image training data (step S304).

The image trainer 131 acquires the image group included in the set, the corresponding additional data (if additional data is included), and the label (step S305), and provides, as input, that image group to the image processor 111 that operates on the basis of the trained image model 151 (step S306).

Then, the image processor 111 and a feature extractor 112 execute image processing (step S307).

Here, the image processor 111 can be implemented in the feature extraction device 101, or may be implemented by referencing the same image model 151 in a device that is independent from the feature extraction device 101.

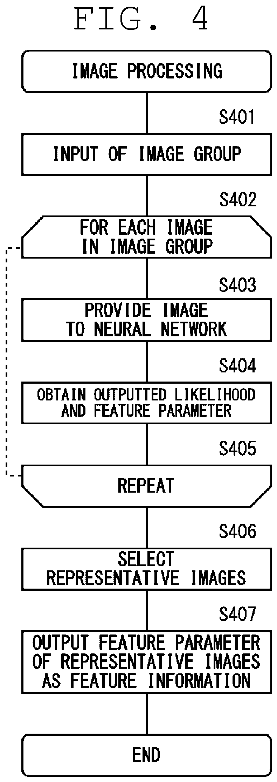

FIG. 4 is a flowchart illustrating the flow of the control of the image processing for obtaining the feature information from the image group. FIG. 4 is referenced in the following description.

When the image processing starts, the image processor 111 receives an input of the image group sequentially, in parallel, or as a batch (step S401), and repeats the following processing for each image included in the inputted image group (step S402).

That is, the image processor 111 provides the images to the neural network related to the image model 151 (step S403), and obtains the likelihoods and the feature parameters output from the neural network (step S404).

When the repeating for all of the images ends (step S405), the feature extractor 112 selects the predetermined number of representative images on the basis of the obtained likelihoods (step S406).

Then, the feature extractor 112 collects and outputs, as the feature information, the feature parameters obtained for the selected representative images (step S407), and ends this processing.

Returning to FIG. 3, the classification trainer 133 acquires the feature information output from the image processor 111 (step S308).

Then, the classification trainer 133 provides, as input into a classifier related to the classification model 153, the acquired feature information and, if input, the additional data (step S309), obtains the result output from the classifier (step S310), and calculates the difference between the outputted result and the label (step S311).

When the repeating for each set ends (step S312), the classification trainer 133 calculates a value of an evaluation function on the basis of the difference calculated for each set and updates the classification model 153 (step S313), and executes the processing of step S302.

When the training of the classification model 153 is complete (step S302; Yes), the classification trainer 133 ends this processing.

Note that the feature extraction and the classification training processings can be executed in parallel at high speed by using a library.

The training of the image model 151 and the classification model 153 may be considered complete when the number of times of repetitions of the updating of the model reaches a predetermined number of times, or may be considered complete when a predetermined convergence condition is satisfied.

### Feature extraction processing

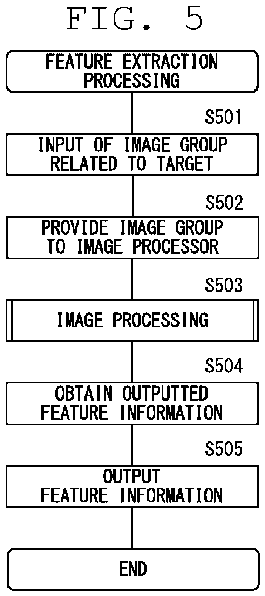

FIG. 5 is a flowchart illustrating the flow of the control of feature extraction processing. FIG. 5 is referenced in the following description.

When this processing starts, the feature extraction device 101 receives an input of an image group related to the target (step S501).

Then, the feature extraction device 101 provides the inputted image group to the image processor 111 (step S502), and causes the image processor 111 and the feature extractor 112 to executes the image processing described above (step S503). Then, the image processor 111 calculates the likelihood and the feature parameter of each image of the image group, and the feature extractor 112 selects the predetermined number of representative images from the image group on the basis of the likelihood, and collects and outputs, as the feature information of the image group, the feature parameters of the representative images.

Then, the feature extraction device 112 acquires the feature information of the image group outputted from the feature processor 111 (step S504).

Then, the feature processor 112 outputs the acquired feature information as feature information related to the target (step S505), and ends this processing.

### Classification processing

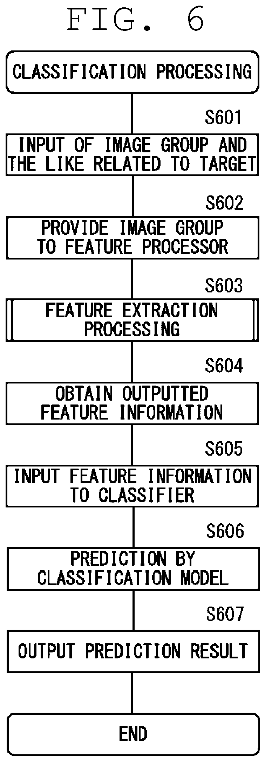

FIG. 6 is a flowchart illustrating the flow of the control of classification processing. FIG. 6 is referenced in the following description.

When this processing starts, the classification processor 113 of the feature extraction device 101 receives an input of the image group related to the target and (if present) the additional data (step S601).

Then, the inputted image group is provided to the feature processor 112 as an input (step S602). Then, the feature processor 112 executes the feature extraction processing described above (step S603).

Then, the classification processor 113 acquires the feature information outputted from the feature processor 112 (step S604), and inputs the acquired feature information and (if input) the additional data thereof into a classifier based on the classification model 153 (step S605).

Then, the classification processor 113 causes the classifier to predict, using the classification model 153 and on the basis of the classification model 153, whether the target belongs to the first class (step S606), outputs the result thereof (step S607), and ends this processing. The outputted result may, in addition to the information indicating whether the target belongs to the first class, also include a probability thereof.

### Experiment results

Hereinafter, experiment results are described for an aspect in which the presence/absence of prostate cancer is predicted using ultrasonic images, according to the present embodiment.

As the data for training and validation, 2,899 ultrasound images for 772 subjects, obtained from November 2017 to June 2020, were prepared.

The size of each image was normalized to 256×256 pixels.

Information about whether each subject is suffering from prostate cancer is attached to each image.

A Gleason score assigned by an expert as a result of observing, under a microscope, a biopsy specimen acquired separately from each subject is associated with each image.

Experiments were carried out for two types of first image classes, namely a method based on whether the subject is suffering from prostate cancer (cancer classification), and a method based on whether the Gleason score assigned to the image is 8 or greater (high-grade cancer classification).

The probability of the image belonging to the first image class was used as the likelihood, and the likelihood was used as the feature parameter. Additionally, for one image group, three representative images were selected in descending order of the absolute value of the difference from 0.5.

The age, the PSA value, the TPV value, and the PSAD value, which are pieces of clinical data, were used as the additional data.

Xception, inceptionV3, and VGG16 were applied for the neural network related to the image model 151.

Three types of models, namely ridge, lasso, and support vector machine (SVM), were used for the classification model 153.

Firstly, regarding the experiment results for the image model 151, Xception demonstrated the best results. Specifically, accuracy of 0.693 (the range of the 95% confidence interval is from 0.640 to 0.746) was obtained in the cancer classification, and accuracy of 0.723 (the range of the 95% confidence interval is from 0.659 to 0.788) was obtained in the high-grade cancer classification.

Next, regarding the experiment results for the classification model 153, SVM demonstrated the best results. Specifically, accuracy of 0.807 (the range of the 95% confidence interval is from 0.719 to 0.894) was obtained in the cancer classification, and accuracy of 0.835 (the range of the 95% confidence interval is from 0.753 to 0.916) was obtained in the high-grade cancer classification.

Note that, when performing classification of the subjects using only clinical data without using the ultrasonic images (prior art), SVM demonstrated the best results with an accuracy of 0.722 (the range of the 95% confidence interval is from 0.620 to 0.824), and it is understood that the accuracy increased dramatically by applying the feature extraction device 101 of the present embodiment.

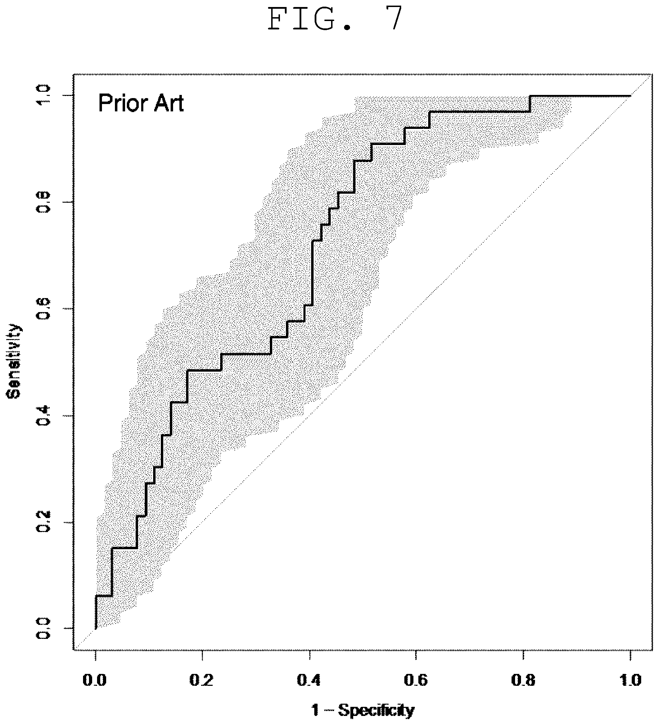

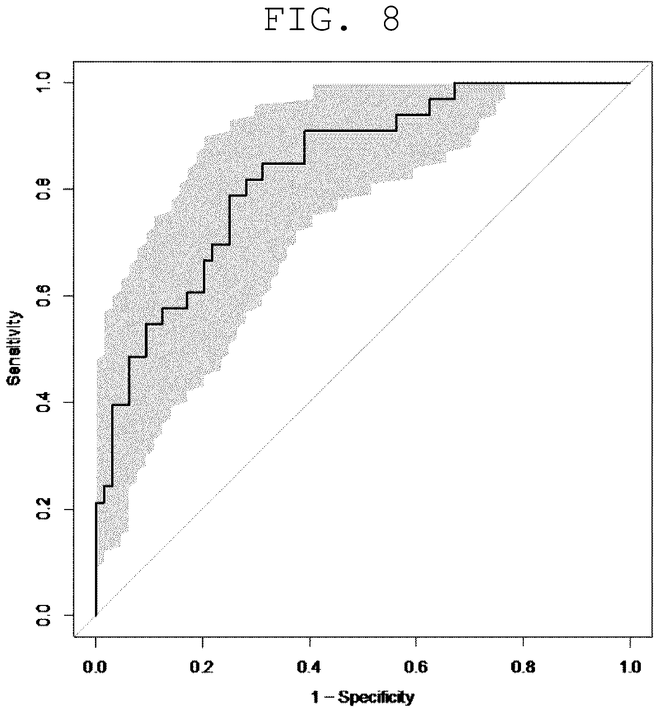

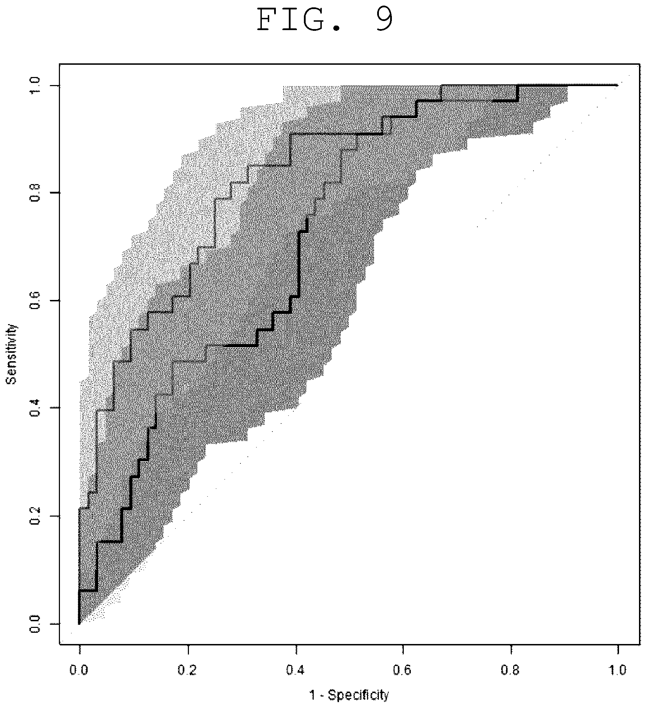

Hereinafter, the prior art and the present embodiment are compared using a receiver operating characteristic (ROC) curve. FIG. 7 is a graph of the experiment results of classification according to a conventional method. FIG. 8 is graph of experiment results of the classification according to the present embodiment. FIG. 9 is an explanatory drawing in which the graph of experiment results of the classification according to the present embodiment and the graph of experiment results of the classification according to the conventional method are stacked and compared. In these drawings, two ROC curves are illustrated, namely an ROC curve of only the clinical data of the conventional method and a ROC curve of the present embodiment. The ROC curve of the present embodiment is moved more to the upper left than the ROC curve of the conventional method, and the area under the curve is greater in the present embodiment than in the conventional method. Accordingly, it is understood that the method according to the present embodiment is more effective than the conventional method.

As a supplemental experiment, classification of targets was attempted using Ridge and Lasso, without the additional data. Upon attempting classifying without selecting the representative images according to the present embodiment, the accuracy was 0.722 and 0.769, respectively but, in the present embodiment, the accuracy was 0.801 and 0.802, respectively. In the feature extraction device 101 according to the present embodiment, it is understood that the accuracy is enhanced as a result of the representative images being selected. It is thought that this is because, in the present embodiment, for subjects with cancer, when the site of the cancer is not captured in an image, that image is not selected as a representative image and, as such, an advantageous effect of reducing noise is obtained.

In the experiments described above, the present embodiment is used to predict, using ultrasound images, the presence/absence of the incidence of prostate cancer. However, as described above, the present embodiment can be applied to diseases other than prostate cancer and images other than ultrasound images.

That is, the present embodiment can be used when extracting features of a subject from multiple images related to that subject to predict whether that subject is suffering from a specific disease or, rather, more broadly and generally, can be used when extracting features of a target from multiple images related to the target and utilizing the features to classify the target.

### Conclusion

As described above, the feature extraction device according to the present embodiment includes:
an image processor that, when an image is input, calculates, using an image model, a likelihood of the inputted image belonging to a first image class, and a feature parameter of the inputted image; and
a feature processor that, when an image group is input, inputs an image included in the inputted image group into the image processor to calculate the likelihood and the feature parameter, selects, based on the calculated likelihood, a predetermined number of representative images from the inputted image group, and outputs, as feature information of the image group, the feature parameter calculated for the selected predetermined number of representative images.

The feature extraction device according to the present embodiment further includes:
a classification processor that, when a target image group related to a target is input, inputs the inputted target image group into the feature processor to predict, from the feature information output from the feature processor and using the classification model, whether the target belongs to a first target class,
wherein
an image included in the target image group related to the target belonging to the first image class corresponds to the target belonging to the first target class.

In the feature extraction device according to the present embodiment,
additional data related to the target is further input into the classification processor, and
the classification processor predicts, from the outputted feature information and the inputted additional data, and using the classification model, whether the target belongs to the first target class.

In the feature extraction device according to the present embodiment,
the target image group includes a plurality of images in which a prostate of the target is captured by ultrasound,
the additional data includes an age, a PSA value, a TPV value, and a PSAD value of the target, and
the first target class is a class that indicates that the target is suffering from prostate cancer.

In the feature extraction device according to the present embodiment, in training data of the image model, the first image class is a class that indicates that, in a biopsy specimen, a Gleason score, assigned to a specimen site corresponding to an image site captured in the image, is greater than or equal to a predetermined value.

In the feature extraction device according to the present embodiment, in training data of the image model, the first image class is a class that indicates that a target related to the image is suffering from prostate cancer.

In the feature extraction device according to the present embodiment, the feature processor selects the predetermined number of representative images in descending order of an absolute value of a difference between the likelihood and a predetermined reference value.

In the feature extraction device according to the present embodiment,
the likelihood is from 0 to 1, and
the predetermined reference value is 0.5.

In the feature extraction device according to the present embodiment, the feature processor selects the predetermined number of representative images in descending order of the likelihood.

In the feature extraction device according to the present embodiment, the feature processor selects, as the predetermined number of representative images, images for which the likelihood is a minimum value, a median value, and a maximum value.

In the feature extraction device according to the present embodiment, the feature parameter calculated for the image is the likelihood calculated for the image.

In the feature extraction device according to the present embodiment, a feature parameter calculated for the image is an intermediate vector of the image in the image model.

In the feature extraction device according to the present embodiment, the image model is a model related to a deep convolutional network.

In the feature extraction device according to the present embodiment, the classification model is a model related to linear regression, logistic regression, ridge regression, lasso regression, or a support vector machine.

A feature extraction method according to the present embodiment includes:
inputting a target image group related to a target into a feature extraction device;
calculating, by the feature extraction device and using an image model, a likelihood of an image included in the inputted target image group belonging to a first image class, and a feature parameter of the image;
selecting, by the feature extraction device and based on the calculated likelihood, a predetermined number of representative images from the inputted target image group; and
outputting, by the feature extraction device and as feature information of the image group, a feature parameter calculated for the selected predetermined number of representative images.

A program according to the present embodiment causes a computer to function as:
an image processor that, when an image is input, calculates, using an image model, a likelihood of the inputted image belonging to a first image class, and a feature parameter of the inputted image; and
a feature processor that, when an image group is input, inputs an image included in the inputted image group into the image processor to calculate the likelihood and the feature parameter, selects, based on the calculated likelihood, a predetermined number of representative images from the inputted image group, and outputs, as a feature of the target, a feature parameter calculated for the selected predetermined number of representative images.

The program is recorded on a non-transitory computer-readable information recording medium according to the present embodiment.

The foregoing describes some example embodiments for explanatory purposes. Although the foregoing discussion has presented specific embodiments, persons skilled in the art will recognize that changes may be made in form and detail without departing from the broader spirit and scope of the invention. Accordingly, the specification and drawings are to be regarded in an illustrative rather than a restrictive sense. This detailed description, therefore, is not to be taken in a limiting sense, and the scope of the invention is defined only by the included claims, along with the full range of equivalents to which such claims are entitled.

This application claims the benefit of Japanese Patent Application No. 2021-089721, filed on Friday, May 28, 2021, the entire disclosure of which is incorporated by reference herein to the extent permitted by the laws of the designated country.

### Industrial Applicability

According to the present disclosure, it is possible to provide a feature extraction device, a feature extraction method, a program, and a non-transitory information recording medium that extract features of a target from a plurality of images related to the target.

### Reference Signs List

- 101: Feature extraction device
- 111: Image processor
- 112: Feature processor
- 113: Classification processor
- 131: Image trainer
- 133: Classification trainer
- 151: Image model
- 153: Classification model

## Claims

1. A feature extraction device comprising:
an image processor that, when an image is input, calculates, using an image model, a likelihood of the inputted image belonging to a first image class, and a feature parameter of the inputted image; and
a feature processor that, when an image group is input, inputs an image included in the inputted image group into the image processor to calculate the likelihood and the feature parameter, selects, based on the calculated likelihood, a predetermined number of representative images from the inputted image group, and outputs, as feature information of the image group, the feature parameter calculated for the selected predetermined number of representative images.

2. The feature extraction device according to claim 1, further comprising:
a classification processor that, when a target image group related to a target is input, inputs the inputted target image group into the feature processor to predict, from the feature information output from the feature processor and using the classification model, whether the target belongs to a first target class,
wherein
an image included in the target image group related to the target belonging to the first image class corresponds to the target belonging to the first target class.

3. The feature extraction device according to claim 2, wherein
additional data related to the target is further input into the classification processor, and
the classification processor predicts, from the outputted feature information and the inputted additional data, and using the classification model, whether the target belongs to the first target class.

4. The feature extraction device according to claim 3, wherein
the target image group includes a plurality of images in which a prostate of the target is captured by ultrasound,
the additional data includes an age, a PSA value, a TPV value, and a PSAD value of the target, and
the first target class is a class that indicates that the target is suffering from prostate cancer.

5. The feature extraction device according to claim 4, wherein in training data of the image model, the first image class is a class that indicates that, in a biopsy specimen, a Gleason score, assigned to a specimen site corresponding to an image site captured in the image, is greater than or equal to a predetermined value.

6. The feature extraction device according to claim 4, wherein in training data of the image model, the first image class is a class that indicates that a target related to the image is suffering from prostate cancer.

7. The feature extraction device according to claim 1, wherein the feature processor selects the predetermined number of representative images in descending order of an absolute value of a difference between the likelihood and a predetermined reference value.

8. The feature extraction device according to claim 7, wherein
the likelihood is from 0 to 1, and
the predetermined reference value is 0.5.

9. The feature extraction device according to claim 1, wherein the feature processor selects the predetermined number of representative images in descending order of the likelihood.

10. The feature extraction device according to claim 1, wherein the feature processor selects, as the predetermined number of representative images, images for which the likelihood is a minimum value, a median value, and a maximum value.

11. The feature extraction device according to claim 1, wherein the feature parameter calculated for the image is the likelihood calculated for the image.

12. The feature extraction device according to claim 1, wherein a feature parameter calculated for the image is an intermediate vector of the image in the image model.

13. The feature extraction device according to claim 1, wherein the image model is a model related to a deep convolutional network.

14. The feature extraction device according to claim 1, wherein the classification model is a model related to linear regression, logistic regression, ridge regression, lasso regression, or a support vector machine.

15. A feature extraction method comprising:
inputting a target image group related to a target into a feature extraction device;
calculating, by the feature extraction device and using an image model, a likelihood of an image included in the inputted target image group belonging to a first image class, and a feature parameter of the image;
selecting, by the feature extraction device and based on the calculated likelihood, a predetermined number of representative images from the inputted target image group; and
outputting, by the feature extraction device and as feature information of the image group, a feature parameter calculated for the selected predetermined number of representative images.

16. A program causing a computer to function as:
an image processor that, when an image is input, calculates, using an image model, a likelihood of the inputted image belonging to a first image class, and a feature parameter of the inputted image; and
a feature processor that, when an image group is input, inputs an image included in the inputted image group into the image processor to calculate the likelihood and the feature parameter, selects, based on the calculated likelihood, a predetermined number of representative images from the inputted image group, and outputs, as a feature of the target, a feature parameter calculated for the selected predetermined number of representative images.

17. A non-transitory computer-readable information recording medium on which the program according to claim 16 is stored.
